# EUROPÄISCHE PATENTSCHRIFT

(11) **EP 3 503 867 B1**
(45) Veröffentlichungstag und Bekanntmachung des Hinweises auf die Patenterteilung: **30.12.2020**
(21) Anmeldenummer: 17742988.3
(22) Anmeldetag: 12.07.2017
(51) Int. Cl.: A61K 8/39, A61K 8/45, A61K 8/86, A61Q 19/10, A61K 8/92

(54) **TRANSPARENTE KOSMETISCHE REINIGUNGSZUSAMMENSETZUNGEN MIT VERBESSERTEM RHEOLOGIEPROFIL**
TRANSPARENT COSMETIC CLEANSING COMPOSITION WITH IMPROVED RHEOLOGICAL PROFIL
COMPOSITION COSMÉTIQUE NÉTTOYANTE AYANT UN PROFIL RHÉOLOGIQUE AMÉLIORÉ

(30) Priorität: 25.08.2016 DE 102016215965
(43) Veröffentlichungstag der Anmeldung: 03.07.2019
(73) Patentinhaber: Henkel AG & Co. KGaA, 40589 Düsseldorf (DE)
(72) Erfinder: SCHELGES, Heike, DE / 47877 Willich (DE); HEIDE, Barbara, 47809 Krefeld (DE); RATHERT, Kristina, 40597 Düsseldorf (DE); SCHOLZ, Elvira, DE / 40764 Langenfeld (DE); PFEIFER, Stephanie, DE / 53773 Hennef (DE); FRANKLIN, Jutta, 42657 Solingen (DE)
(86) Internationale Anmeldenummer: PCT/EP2017/067589
(87) Internationale Veröffentlichungsnummer: WO 2018/036717

(56) Entgegenhaltungen:
- EP-B- 2 296 619
- WO-A1-2016/034519
- DE-A1- 10 161 885
- DE-A1-102011 078 111
- US-A- 5 192 462
- US-A1- 2002 037 267
- US-A1- 2003 092 586

## Beschreibung

Die Erfindung liegt auf dem Gebiet der Kosmetik und betrifft transparente Reinigungszusammensetzungen mit hoher Pflegeleistung und gutem Rheologieprofil, die neben Tensiden, Wasser und Ölen eine spezifische Wirkstoffmischung aus Lösungsvermittlern und Verdickungsmitteln enthalten.

Kosmetische Reinigungszusammensetzungen enthalten üblicherweise Tenside. Aufgrund ihrer hervorragenden Schaumeigenschaften werden als Primärtenside meist anionische Tenside eingesetzt, die jedoch in höheren Mengen den Nachteil aufweisen können, dass sie die Haut aufweichen und Lipide aus den äußeren Hautschichten entfernen. Dadurch kann die (empfindliche) Haut insbesondere nach häufiger Reinigung mit handelsüblichen Reinigungsmitteln trocken, spröde und mitunter rissig werden, sofern ihr nicht nach oder während der Reinigung Lipide in der Form von Cremes, Lotionen und/oder Pflegeduschbädern wieder zugeführt werden.

In der Vergangenheit wurden zahlreiche Versuche unternommen, mildere, pflegende kosmetische Reinigungsmittel herzustellen, welche die Haut weniger austrocknen und einen weiteren Behandlungsschritt mit Pflegemitteln entbehrlich werden lassen.
Die mangelnde Stabilität milder Zusammensetzungen, welche oftmals einen hohen Anteil konditionierender Komponenten und/oder einen niedrigen Anteil an (anionischen) Tensiden enthalten, ist jedoch nach wie vor problematisch.
Darüber hinaus werden von Verbrauchern oftmals klare Zusammensetzungen bevorzugt, weil sie mit solchen Produkten neben der Reinigung außerdem ein intensiveres Frische - und/oder Feuchtigkeitsgefühl assoziieren.
Die Formulierung transparenter, milder Reinigungszusammensetzungen bereitet zusätzliche formulierungstechnische Probleme, da die effiziente Solubilisierung von ölhaltigen Konditionierungsmitteln oftmals zu Lasten des Schaumverhaltens und/oder zu einem unerwünschten Viskositätsverlust von Reinigungszusammensetzungen führen kann.

In der Anmeldung WO 2003/051319A1 wird ein Verfahren zur Herstellung ölhaltiger Mikroemulsionen offenbart, bei dem flüssige und/oder feste Öle mithilfe von W/O-Emulgatoren, Verdickungsmitteln und Salzen in Primär- und Co-Tenside enthaltenden Zusammensetzungen stabilisiert werden.

Die gemäß diesem Verfahren herstellbaren Mikroemulsionen weisen jedoch den Nachteil auf, dass sie niedrigviskos und damit für die Verwendung als klassische kosmetische Reinigungsprodukte wie beispielsweise Duschgele nicht uneingeschränkt geeignet sind.

Darüber hinaus bleibt nach der Anwendung der Mikroemulsionen ein Lipidfilm auf der Haut haften, der durch die zusätzliche Verwendung von Wachsen noch verstärkt wird.

In der Patentanmeldung DE102011078111 wird ein Reinigungsmittel offenbart, das in einem kosmetischen akzeptablen Träger ein pflanzliches Öl, ein ethoxyliertes Glycerylester, und ethoxyliertes hydriertes Rizinusöl mit einem mittleren Ethoxylierungsgrad enthält.

Der vorliegenden Erfindung lag die Aufgabe zugrunde, milde, pflegende kosmetische Reinigungszusammensetzungen hoher Transparenz und Stabilität zur Verfügung zu stellen.
Die transparenten Reinigungszusammensetzungen sollten eine ausreichende Viskosität für die bequeme Applikation aus einem üblichen Duschgelbehältnis sowie ein gutes Rheologieprofil aufweisen.
Ferner sollten die transparenten Reinigungszusammensetzungen der Haut Feuchtigkeit spenden und ein weiches, gepflegtes Hautgefühl hinterlassen. Insbesondere sollte nach dem Abspülen der Zusammensetzungen kein Ölfilm auf der Haut verbleiben.
Es wurde überraschend gefunden, dass die zuvor genannten Aufgaben in hervorragendem Maße mithilfe kosmetischer Reinigungszusammensetzungen gelöst werden, in denen Öle mithilfe spezieller Lösungsvermittler und Verdickungsmittel in einer Wasser und Tenside enthaltenden Basis stabilisiert wurden.
Die Reinigungszusammensetzungen sind transparent, viskos (d.h., dass sie bevorzugt eine Viskosität im Bereich von 2,000 bis 15,000 mPas, mehr bevorzugt von 3,000 bis 11,000 mPas und insbesondere von 4,000 bis 9,000 mPas aufweisen, wobei die Viskosität mit einem Haake Visco Tester 550 MV II, 7,2 S-1 bei 20°C bestimmt wurde), lagerstabil, und sie weisen ein hervorragendes Fließverhalten (Öl-analoges, newtonsches Rheologieprofil) auf.
Darüber hinaus hinterlassen sie nach ihrer Anwendung ein nicht-öliges, glattes, trockenes Hautgefühl und erhalten insbesondere die Feuchtigkeitsbalance der Haut während und nach der Reinigung.
Ein erster Gegenstand dieser Anmeldung ist eine transparente kosmetische Reinigungszusammensetzung, enthaltend neben mindestens einem Tensid und Wasser:
a) mindestens ein Öl,
b) mindestens einen Polyethylengruppen enthaltenden Lösungsvermittler, ausgewählt aus
   - Anlagerungsprodukten von 1 bis 20 Mol Ethylenoxid an Mono-, Di- und/oder Triester von Glycerin und linearen oder verzweigten, gesättigten oder ungesättigten C₈-C₂₄-Carbonsäuren in einem Gewichtsanteil von 0,1 bis 3,0 Gew.-% am Gesamtgewicht der Reinigungszusammensetzung und/oder
   - Anlagerungsprodukten von 25 bis 80 Mol Ethylenoxid an Rizinusöl und/oder
   gehärtetes Rizinusöl, in einem Gewichtsanteil von 0,5 bis 5,0 Gew.-% am Gesamtgewicht der Reinigungszusammensetzung, und
c) mindestens ein Polyethylen- und/oder Polypropylengruppen enthaltendes Verdickungsmittel, in einem Gewichtsanteil von 0,01 bis 2,00 Gew.-% am Gesamtgewicht der Reinigungszusammensetzung, ausgewählt aus
   - Anlagerungsprodukten von 1 bis 10 Mol Propylenoxid an C₈-C₂₄-Carbonsäureamide und/oder
   - Anlagerungsprodukten von 100 bis 300 Mol Ethylenoxid und/oder Propylenoxid an Mono-, Di-, Tri- und/oder Tetraester von Pentaerythrit an lineare oder verzweigte, gesättigte oder ungesättigte C₈-C₂₄-Carbonsäuren.

Unter "Transparenz" im Sinne der vorliegenden Erfindung wird ein Trübungswert der erfindungsgemäßen Zusammensetzungen von maximal 150 NTU, mehr bevorzugt von maximal 100 NTU und insbesondere von maximal 50 NTU verstanden.
Die Transparenz einer Zusammensetzung kann nach bekannten Verfahren durch ihre Trübung bestimmt werden, wobei der ermittelte NTU-Wert (nephelometric turbidity unit) das Maß der Trübung angibt.
Trübungsmessungen können beispielsweise mit einem Turbidimeter (beispielsweise der Firma Hach) bei 20°C bis 25°C durchgeführt werden.

Die erfindungsgemäßen Reinigungszusammensetzungen können in einer bevorzugten Ausführungsform als flüssige Reinigungszusammensetzungen wie beispielsweise Flüssigseifen, Duschgele, Duschcremes, Duschbäder, Duschbalsame und/oder Cremebäder konfektioniert werden. In diesem Fall enthalten sie die Wirkstoffe a) bis c) bevorzugt in einem geeigneten kosmetischen Träger. Darunter wird im Rahmen der Erfindung bevorzugt ein wässriger Träger verstanden, der (bezogen auf das Gesamtgewicht der kosmetischen Reinigungszusammensetzungen) vorzugsweise mindestens 50 Gew.-%, mehr bevorzugt mindestens 55 Gew.-%, besonders bevorzugt mindestens 60 Gew.-% und insbesondere bevorzugt mindestens 65 Gew.-% Wasser enthält.
Weiterhin kann der kosmetische Träger 0,01 bis 40 Gew.-%, bevorzugt 0,05 bis 30 Gew.-% und insbesondere 0,1 bis 20 Gew.-% mindestens eines Alkohols enthalten.
Geeignete Alkohole sind beispielsweise Ethanol, Ethyldiglykol, 1-Propanol, 2-Propanol, Isopropanol, 1,2-Propylenglycol, 1,3-Propylenglycol, 1-Butanol, 2-Butanol, 1,2-Butandiol, 1,3-Butandiol, 1,4-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Glycerin, Polyethylenglycol, Sorbitol, Sorbitan, Benzylalkohol, Phenoxyethanol oder Mischungen dieser Alkohole.
Bevorzugt sind die wasserlöslichen Alkohole wie Ethanol, 1,2-Propylenglycol, Glycerin, Sorbitol, Benzylalkohol und/oder Phenoxyethanol sowie Mischungen dieser Alkohole.
Insbesondere bevorzugt sind Glycerin und/oder Sorbitol.

In einer besonders bevorzugten Ausführungsform enthalten die erfindungsgemäßen Reinigungszusammensetzungen einen wässrig-alkoholischen Träger, der vorzugsweise mindestens 5 Gew.-%, mehr bevorzugt mindestens 6 Gew.-% und insbesondere mindestens 7,5 Gew.-% mindestens eines Alkohols, vorzugsweise Glycerin und/oder Sorbitol enthält.

Ein Gehalt an Glycerin und/oder Sorbitol trägt nicht nur zur besseren Löslichkeit der Ölkomponente(n) in den erfindungsgemäßen Zusammensetzungen bei, sondern erhöht auch deren Pflegewirkung auf der Haut.

Als milde und gut schäumende Tensidbasis für die erfindungsgemäßen Reinigungsmittel hat sich vorzugsweise eine Mischung aus einem oder mehreren anionischen Haupttensiden und mindestens einem amphoteren Co-Tensid herausgestellt.

In einer ersten bevorzugten Ausführungsform enthalten die erfindungsgemäßen Reinigungszusammensetzungen daher mindestens ein anionisches Tensid (i), bevorzugt eine Mischung aus mindestens einem anionischen (i) und mindestens einem amphoteren / zwitterionischen Tensid (ii).

Besonders gute Ergebnisse im Hinblick auf eine optimale Balance zwischen Milde und guten Schaumeigenschaften der erfindungsgemäßen kosmetischen Reinigungszusammensetzungen können durch
- sorgfältige Auswahl der Art der Tenside (i) und (ii),
- die Gesamtmenge der Tenside (i) + (ii) und
- das Gewichtsverhältnis der Tenside (i) : (ii) noch weiter verbessert werden.
Es hat sich herausgestellt, dass die Gesamtmenge an anionischen und amphoteren Tensiden (i) + (ii) in den erfindungsgemäßen Zusammensetzungen bevorzugt mindestens 8,0 Gew.-%, mehr bevorzugt mindestens 9,0 Gew.-% und insbesondere mindestens 10,0 Gew.-% betragen sollte (bezogen auf das Gesamtgewicht der erfindungsgemäßen Reinigungszusammensetzungen). Weiterhin hat sich herausgestellt, dass ein Gewichtsverhältnis der Tenside (i) : (ii) im Bereich von 2 : 1 bis 1 : 1 zu besonders milden Reinigungszusammensetzungen führt.

In einer weiteren bevorzugten Ausführungsform sind daher erfindungsgemäße Reinigungszusammensetzungen bevorzugt, in denen die Gesamtmenge an anionischen und amphoteren Tensiden (i) + (ii) mindestens 8,0 Gew.-%, mehr bevorzugt mindestens 9,0 Gew.-% und insbesondere mindestens 10,0 Gew.-% beträgt und/oder in denen das Gewichtsverhältnis der Tenside (i) : (ii) im Bereich von 2 : 1 bis 1 : 1 liegt.
Besonders bevorzugt innerhalb dieser Ausführungsform sind erfindungsgemäße Reinigungszusammensetzungen in denen das Gewichtsverhältnis der Tenside (i) : (ii) im Bereich von 2 : 1 bis 1 1 liegt, und insbesondere bevorzugt sind erfindungsgemäße Reinigungszusammensetzungen, in denen beide der zuvor genannten Bedingungen erfüllt sind.

Bevorzugte anionische Tenside (i) im Sinne der vorliegenden Erfindung können ausgewählt sein aus
- Acylsarcosinaten mit 8 bis 24 C-Atomen in der Acylgruppe,
- Acyltauraten mit 8 bis 24 C-Atomen in der Acylgruppe,
- Acylisethionaten mit 8 bis 24 C-Atomen in der Acylgruppe,
- Acylglycinaten mit 8 bis 24 C-Atomen in der Acylgruppe,
- Acyllactylaten mit 8 bis 24 C-Atomen in der Acylgruppe,
- Sulfobernsteinsäuremono- und/oder -dialkylestern mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen, und/oder
- Alkylsulfaten und/oder Alkylethersulfatsalzen der Formel R-(OCH₂-CH₂)ₙ-O-SO₃X, in der R bevorzugt eine geradkettige oder verzweigte, gesättigte oder ungesättigte Alkylgruppe mit 8 bis 24 C-Atomen, x die Zahl 0 oder 1 bis 12 und X ein Alkali-, Erdalkali-, Ammonium- oder Alkanolaminion bedeutet.

Besonders bevorzugte anionische Tenside (i) sind Alkali-, Erdalkali- und/oder Ammoniumsalze von linearen oder verzweigten, gesättigten oder ungesättigten Alkylsulfaten, Alkylethersulfaten, Acylisethionaten, Acylglycinaten, Acyltauraten, Acylsarcosinaten, Acyllactylaten und/oder Acylsulfosuccinaten, deren Alkyl- oder Acylgruppen jeweils 6 bis 24 Kohlenstoffatome enthalten. Insbesondere bevorzugt sind die unter den INCI-Bezeichnungen Sodium Laureth Sulfate, Sodium Myreth Sulfate, Sodium Trideceth Sulfate, Sodium Laureth Sulfosuccinate, Sodium Lauroyl Lactylate, Sodium Stearoyl Lactylate, Sodium Cocoyl Isethionate, Sodium Methyl Cocoyl Isethionate, Sodium Cocoyl Glycinate, Sodium Methyl Cocoyl Glycinate, Sodium Cocoyl Taurate, Sodium Methyl Cocoyl Taurate, Sodium Myristoyl Sarcosinate und/oder Sodium Cocoyl Sarcosinate bekannten Verbindungen, sowie deren Mischungen.

Der Gewichtsanteil des mindestens einen anionischen Tensids (i) am Gesamtgewicht der erfindungsgemäßen Zusammensetzungen beträgt bevorzugt 5,00 bis 15,0 Gew.-%, mehr bevorzugt 5,5 bis 12,5 Gew.-%, besonders bevorzugt von 6,0 bis 11,0 Gew.-% und insbesondere in Mengen von 6,5 bis 10,0 Gew.-%.

Zu den geeigneten amphoteren Tensiden (ii), die in den erfindungsgemäßen Reinigungszusammensetzungen eingesetzt werden können, zählen beispielsweise eine oder mehrere Verbindungen der nachfolgenden Formeln (I) bis (VII), in denen der Rest R jeweils für einen geradkettigen oder verzweigten, gesättigten oder ein- oder mehrfach ungesättigten Alkyl- oder Alkenylrest mit 7 bis 23 Kohlenstoffatomen (Formeln (I) und (II)) oder für einen geradkettigen oder verzweigten, gesättigten oder ein- oder mehrfach ungesättigten Alkyl- oder Alkenylrest mit 8 bis 24 Kohlenstoffatomen (Formeln (III) bis (VII)) steht:

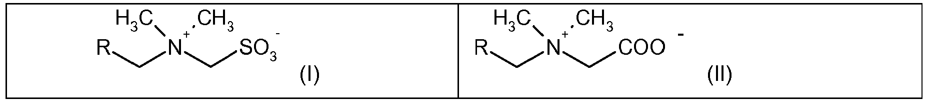

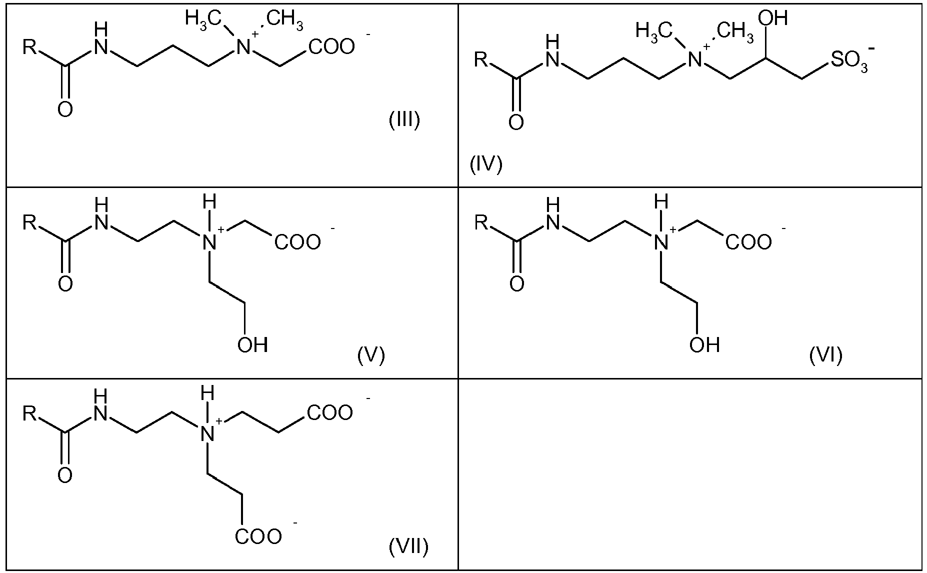

Bevorzugte amphotere Tenside einer der zuvor genannten Formeln (I) bis (VII) enthalten als Rest R überwiegend einen geradkettigen oder verzweigten, gesättigten, ein- oder mehrfach ungesättigten Alkylrest mit 8 bis 24, mehr bevorzugt von 8 bis 18 und insbesondere mit 8 bis 16 C-Atomen. Mehr bevorzugt sind C₈-C₂₄-Alkylbetaine, C₈-C₂₄-Alkylamido-(C₂-C₄)-alkyl-betaine, C₈-C₂₄-Fettsäureamphoacetate und/oder -propionate, wobei es besonders bevorzugt ist, wenn sich der Rest R von Kokosfett ableitet.
Ganz besonders die unter den INCI-Bezeichnungen Sodium Cocoamphoacetate, Disodium Cocoamphodiacetate, Sodium Lauroamphoacetate, Sodium Lauroamphodiacetate, Sodium Cocoamphopropionate, Disodium Cocoamphodipropionate, Coco Betaine, Lauryl Betaine Cocamidopropylbetain und/oder Lauramidopropylbetain bekannten und im Handel von mehreren Anbietern erhältlichen amphoteren/zwitterionischen Tenside eignen sich für die Verwendung in den erfindungsgemäßen Zusammensetzungen, wobei Tenside mit der INCI-Bezeichnung Cocamidopropylbetain insbesondere bevorzugt sind.

Der Gewichtsanteil des mindestens einen amphoteren Tensids (ii) am Gesamtgewicht der erfindungsgemäßen Reinigungszusammensetzungen beträgt bevorzugt 2,0 bis 15,0 Gew.-%, mehr bevorzugt 2,5 bis 10,0 Gew.-%, besonders bevorzugt von 3,0 bis 9,0 Gew.-% und insbesondere in Mengen von 3,5 bis 8,0 Gew.-%.

Eine zweite besonders bevorzugte Ausführungsform der Erfindung ist dadurch gekennzeichnet, dass sie
- ein oder mehrere anionische Tenside (i) in einem Gewichtsanteil von 5,0 bis 15,0 Gew.-% am Gesamtgewicht der Reinigungszusammensetzung und/oder
- eine Mischung mindestens einem anionischen (i) und mindestens einem amphoteren / zwitterionischen Tensid (ii) in einem Gewichtsanteil von 7,0 bis 20,0 Gew.-% am Gesamtgewicht der Reinigungszusammensetzung enthält.

Eine weitere wesentliche Komponente der erfindungsgemäßen Zusammensetzungen ist ein Öl a).

Erfindungsgemäß geeignete Öle können aus natürlichen (pflanzlichen), synthetischen und/oder mineralischen Ölen ausgewählt sein, wobei Öle natürlichen (pflanzlichen) Ursprungs aufgrund ihrer hervorragenden Hautpflegewirkung sowie aus Gründen ihrer Bioverfügbarkeit bevorzugt sind. Unter natürlichen (pflanzlichen) Ölen sind erfindungsgemäß Parfumöle und/oder pflanzliche Triglyceridöle, wie beispielsweise Kokosöl, (süßes) Mandelöl, Walnussöl, Pfirsichkernöl, Aprikosenkernöl, Avocadoöl, Teebaumöl (Tea Tree Oil), Sojaöl, Baumwollsaatöl, Sesamöl, Sonnenblumenöl, Tsubakiöl, Nachtkerzenöl, Reiskleieöl, Palmöl, Palmkernöl, Mangokernöl, Cranberryöl, Sanddornöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Rizinusöl, Safloröl, Maisöl, Olivenöl, Rapsöl, Canolaöl, Sasanquaöl, Jojobaöl, Rambutanöl, Kakaobutter und/oder Shea-Butter zu verstehen.

Der Gewichtsanteil des mindestens einen pflanzlichen Öls a) am Gesamtgewicht der erfindungsgemäßen Reinigungszusammensetzungen beträgt bevorzugt 0,1 bis 2,0 Gew.-%, mehr bevorzugt 0,2 bis 1,9 Gew.-%, besonders bevorzugt 0,3 bis 1,8 Gew.-% und insbesondere 0,4 bis 1,7 Gew.-%.

Die Einarbeitung schwer löslicher Parfümöle in Reinigungszusammensetzungen, die eine hohe Transparenz und Lagerstabilität aufweisen sollen, ist problematisch.
Insbesondere für den Fall, dass höhere Parfumölmengen oder Kombinationen von Parfumölen und Triglyceriden eingesetzt werden sollen, bereitet die Auswahl eines geeigneten Solubilisierungsmittels (Lösungsvermittlers) mitunter Probleme.

Es wurde gefunden, dass sich sowohl höhere Parfumölmengen als auch Kombinationen von Parfumölen und Triglyceriden in zuvor beschriebene Tensidmischungen klar und stabil einarbeiten lassen, wenn als Solubilisierungsmittel (Lösungsvermittler b)) für das oder die Öl(e)
- Anlagerungsprodukte von 1 bis 20 Mol Ethylenoxid an Mono-, Di- und/oder Triester von Glycerin und linearen oder verzweigten, gesättigten oder ungesättigten C₈-C₂₄-Carbonsäuren und/oder
- Anlagerungsprodukte von 25 bis 80 Mol Ethylenoxid an Rizinusöl und/oder gehärtetes Rizinusöl
eingesetzt werden.

Zur Erzielung der optimaler Transparenz und Stabilität der erfindungsgemäßen Zusammensetzungen ist es besonders bevorzugt, wenn eine Kombination der beiden zuvor genannten Solubilisierungstypen eingesetzt wird.

Eine dritte bevorzugte Ausführungsform der Erfindung ist demnach dadurch gekennzeichnet, dass die Reinigungszusammensetzungen
- Anlagerungsprodukte von 1 bis 20 Mol Ethylenoxid an Mono-, Di- und/oder Triester von Glycerin und linearen oder verzweigten, gesättigten oder ungesättigten C₈-C₂₄-Carbonsäuren in einem Gewichtsanteil von 0,1 bis 3,0 Gew.-% am Gesamtgewicht der Reinigungszusammensetzung, und
- Anlagerungsprodukte von 25 bis 80 Mol Ethylenoxid an Rizinusöl und/oder gehärtetes Rizinusöl in einem Gewichtsanteil von 0,5 bis 5,0 Gew.-% am Gesamtgewicht der Reinigungszusammensetzung enthalten.

Besonders bevorzugte Solubilisierungsmittel (Lösungsvermittler b)) der zuvor genannten Gruppen sind die unter den INCI-Bezeichnungen bekannten Verbindungen:
PEG-7 Glyceryl Oleate (HLB 10); PEG-25 Hydrogenated Castor Oil (HLB 10,8); PEG-40 Hydrogenated Castor Oil (HLB 14) und PEG-60 Hydrogenated Castor Oil (HLB 16).

Eine weitere bevorzugte Ausführungsform ist dadurch gekennzeichnet, dass als Lösungsvermittler b)
- eine unter der INCI-Bezeichnung PEG-7 Glyceryl Cocoate bekannte Verbindung in einem Gewichtsanteil von 0,1 bis 3,0 Gew.-% am Gesamtgewicht der Reinigungszusammensetzung und
- eine unter der INCI-Bezeichnung PEG-40 Hydrogenated Castor Oil und/oder eine unter der INCI-Bezeichnung PEG-60 Hydrogenated Castor Oil bekannte Verbindung (jeweils) in einem Gewichtsanteil von 0,5 bis 5,0 Gew.-% am Gesamtgewicht der Reinigungszusammensetzung eingesetzt wird.

Neben der Transparenz sollen die erfindungsgemäßen Zusammensetzungen auch ein gutes Rheologieprofil aufweisen und die bequeme Applikation aus einem üblichen Duschgelbehältnis gewährleisten.
Die vollständige und stabile Solubilisierung von Parfumölen und/oder Triglyceridölen mit den Lösungsvermittlern b) gelingt problemlos, doch die resultierenden Reinigungszubereitungen weisen eine unerwünschte, sehr niedrige bis wasserdünne Viskosität auf, die sich nicht - wie bei Tensidlösungen üblich - mit der Zugabe von Elektrolytsalzen erhöhen lässt.

Auch die Einarbeitung polymerer Verdickungsmittel auf Acrylsäurebasis und/oder natürlicher Polysaccharid-Verdickungsmittel reicht nicht aus, um Reinigungszusammensetzungen mit dem gewünschten Rheologieprofil herzustellen. Vielmehr führt die Zugabe polymerer Verdickungsmittel auf Acrylsäurebasis und/oder natürlicher Polysaccharid-Verdickungsmittel zu einer Gelierung der Produkte, die allzu starr und nicht mehr ausreichend fließfähig sind.

Es wurde gefunden, dass Reinigungszusammensetzungen mit dem gewünschten Rheologieprofil sowie hoher Transparenz und Stabilität zur Verfügung gestellt werden können, wenn als Verdickungsmittel bestimmte Polyethylen- und/oder Polypropylengruppen enthaltende Verbindungen c) eingesetzt werden.

Eine weitere wesentliche Komponente der erfindungsgemäßen Zusammensetzungen ist demnach mindestens ein Polyethylen- und/oder Polypropylengruppen enthaltendes Verdickungsmittel c), das ausgewählt aus
- Anlagerungsprodukten von 1 bis 10 Mol Propylenoxid an C₈-C₂₄-Carbonsäureamide und/oder
- Anlagerungsprodukten von 100 bis 300 Mol Ethylenoxid und/oder Propylenoxid an Mono-, Di-, Tri- und/oder Tetraester von Pentaerythrit an lineare oder verzweigte, gesättigte oder ungesättigte C₈-C₂₄-Carbonsäuren.

Das oder die Polyethylen- und/oder Polypropylengruppen enthaltende(n) Verdickungsmittel c) wird in den erfindungsgemäßen Reinigungszusammensetzungen bevorzugt in einem Gewichtsanteil von 0,01 bis 2,00 Gew.-%, mehr bevorzugt von 0,03 bis 1,75 Gew.-%, besonders bevorzugt von 0,05 bis 1,50 Gew.-% und insbesondere von 0,075 bis 1,25 Gew.-% am Gesamtgewicht der Reinigungszusammensetzungen eingesetzt.

Erfindungsgemäß geeignete Anlagerungsprodukte von 1 bis 10 Mol Propylenoxid an C₈-C₂₄-Carbonsäureamide werden bevorzugt aus den unter den INCI-Bezeichnungen PPG-(1-3) Hydroxyethyl Caprylamide, PPG-(1-3) Hydroxyethyl Decylamide, PPG-(1-3) Hydroxyethyl Laurylamide, PPG-(1-3) Hydroxyethyl Myristylamide, PPG-(1-3) Hydroxyethyl Cocamide, PPG-(1-3) Hydroxyethyl Cetylamide, PPG-(1-3) Hydroxyethyl Stearylamide, PPG-(1-3) Hydroxyethyl Isostearylamide, PPG-(1-3) Hydroxyethyl Linoleylamide und/oder PPG-(1-3) Hydroxyethyl Oleylamide bekannten Verbindungen ausgewählt.
Besonders bevorzugt sind PPG-1 Hydroxyethyl Caprylamide, PPG-2 Hydroxyethyl Cocamide und/oder PPG-3 Hydroxyethyl Linoleylamide und insbesondere bevorzugt ist PPG-2 Hydroxyethyl Cocamide.
Entsprechende Produkte sind bekannt und im Handel von verschiedenen Anbietern erhältlich, beispielsweise unter der Handelsbezeichnung "Promidium®" von der Firma Uniqema. Erfindungsgemäß geeignete Anlagerungsprodukte von 100 bis 300 Mol Ethylenoxid und/oder Propylenoxid an Mono-, Di-, Tri- und/oder Tetraester von Pentaerythrit an lineare oder verzweigte, gesättigte oder ungesättigte C₈-C₂₄-Carbonsäuren werden bevorzugt aus den unter den INCI-Bezeichnungen PEG-105 Pentaerythrityl monobehenate, PEG-105 Pentaerythrityl dibehenate, PEG-105 Pentaerythrityl tribehenate, PEG-105 Pentaerythrityl tetrabehenate, PEG-150 Pentaerythrityl monoisostearate, PEG-150 Pentaerythrityl diisostearate, PEG-150 Pentaerythrityl triisostearate, PEG-150 Pentaerythrityl tetraisostearate, PEG-130 Pentaerythrityl monostearate, PEG-130 Pentaerythrityl distearate, PEG-130 Pentaerythrityl tristearate, PEG-150 Pentaerythrityl Monostearate, PEG-150 Pentaerythrityl Distearate, PEG-150 Pentaerythrityl Tristearate, PEG-150 Pentaerythrityl Tetrastearate, PEG-150 Pentaerythrityl monolaurate, PEG-150 Pentaerythrityl dilaurate, PEG-150 Pentaerythrityl trilaurate und/oder PEG-150 Pentaerythrityl tetralaurate bekannten Verbindungen.
Besonders bevorzugt ist PEG-150 Pentaerythrityl Tetrastearate.
Entsprechende Produkte sind bekannt und im Handel von verschiedenen Anbietern erhältlich, beispielsweise unter den Handelsbezeichnungen "Crothix®" oder "Versathix® LQ" von der Firma Croda.

Die zuvor genannten Verdickungsmitteltypen c) - insbesondere die unter den INCI-Bezeichnungen PPG-2 Hydroxyethyl Cocamide und PEG-150 Pentaerythrityl Tetrastearate bekannten Verbindungen - können in den erfindungsgemäßen Reinigungszusammensetzungen sowohl einzeln als auch in Kombination eingesetzt werden.
Ein besonders gutes Rheologie- und Fließverhalten bei stabiler Viskosität und Transparenz kann erzielt werden, wenn eine Kombination der unter den INCI-Bezeichnungen PPG-2 Hydroxyethyl Cocamide und PEG-150 Pentaerythrityl Tetrastearate bekannten Verdickungsmitteltypen c) in den erfindungsgemäßen Reinigungszusammensetzungen eingesetzt wird.

Eine vierte bevorzugte Ausführungsform der Erfindung ist dadurch gekennzeichnet, dass die Reinigungszusammensetzungen als Polyethylen- und/oder Polypropylengruppen enthaltendes Verdickungsmittel c)
- mindestens eine der unter den INCI-Bezeichnungen PEG-105 Pentaerythrityl monobehenate, PEG-105 Pentaerythrityl dibehenate, PEG-105 Pentaerythrityl tribehenate, PEG-105 Pentaerythrityl tetrabehenate, PEG-150 Pentaerythrityl monoisostearate, PEG-150 Pentaerythrityl diisostearate, PEG-150 Pentaerythrityl triisostearate, PEG-150 Pentaerythrityl tetraisostearate, PEG-130 Pentaerythrityl monostearate, PEG-130 Pentaerythrityl distearate, PEG-130 Pentaerythrityl tristearate, PEG-150 Pentaerythrityl tetrastearate, PEG-150 Pentaerythrityl monolaurate, PEG-150 Pentaerythrityl dilaurate, PEG-150 Pentaerythrityl trilaurate und/oder PEG-150 Pentaerythrityl tetralaurate bekannten Verbindungen, und/oder
- mindestens eine der unter den INCI-Bezeichnungen PPG-2 Hydroxyethyl Cocamide, PPG-1 Hydroxyethyl Caprylamide und/oder PPG-3 Hydroxyethyl Linoleamide bekannten Verbindungen enthalten.

Innerhalb dieser Ausführungsform ist es besonders bevorzugt, wenn die Reinigungszusammensetzungen als Polyethylen- und/oder Polypropylengruppen enthaltendes Verdickungsmittel c)
- mindestens eine unter der INCI-Bezeichnung PEG-150 Pentaerythrityl Tetrastearate bekannte Verbindung oder
- mindestens eine unter der INCI-Bezeichnung PPG-2 Hydroxyethyl Cocamide bekannte Verbindung enthalten.

Innerhalb dieser Ausführungsform ist es weiterhin besonders bevorzugt, wenn die Reinigungszusammensetzung als Polyethylen- und/oder Polypropylengruppen enthaltendes Verdickungsmittel c)
- mindestens eine unter der INCI-Bezeichnung PEG-150 Pentaerythrityl Tetrastearate bekannte Verbindung und
- mindestens eine unter der INCI-Bezeichnung PPG-2 Hydroxyethyl Cocamide bekannte Verbindung enthalten.

Eine ganz besonders bevorzugte Ausführungsform der Erfindung ist eine transparente Reinigungszusammensetzung, die
- 7,0 bis 20,0 Gew.-% einer Mischung aus mindestens einem anionischen (i) und mindestens eines amphoteren / zwitterionischen Tensid (ii),
- 0,1 bis 2,0 Gew.-% mindestens eines Öls, vorzugsweise eines Parfumöls und/oder eines pflanzlichen Öls,
- 0,1 bis 3,0 Gew.-% mindestens eines Anlagerungsproduktes von 1 bis 20 Mol Ethylenoxid an Mono-, Di- und/oder Triester von Glycerin und linearen oder verzweigten, gesättigten oder ungesättigten C₈-C₂₄-Carbonsäuren,
- 0,5 bis 5,0 Gew.-% mindestens eines Anlagerungsproduktes von 25 bis 80 Mol Ethylenoxid an Rizinusöl und/oder gehärtetes Rizinusöl,
- 0,01 bis 2,00 Gew.-% mindestens einer der unter den INCI-Bezeichnungen PEG-105 Pentaerythrityl monobehenate, PEG-105 Pentaerythrityl dibehenate, PEG-105 Pentaerythrityl tribehenate, PEG-105 Pentaerythrityl tetrabehenate, PEG-150 Pentaerythrityl monoisostearate, PEG-150 Pentaerythrityl diisostearate, PEG-150 Pentaerythrityl triisostearate, PEG-150 Pentaerythrityl tetraisostearate, PEG-130 Pentaerythrityl monostearate, PEG-130 Pentaerythrityl distearate, PEG-130 Pentaerythrityl tristearate, PEG-150 Pentaerythrityl tetrastearate, PEG-150 Pentaerythrityl monolaurate, PEG-150 Pentaerythrityl dilaurate, PEG-150 Pentaerythrityl trilaurate und/oder PEG-150 Pentaerythrityl tetralaurate bekannten Verbindungen, und
- 0,01 bis 2,00 Gew.-% mindestens einer der unter den INCI-Bezeichnungen PPG-2 Hydroxyethyl Cocamide, PPG-1 Hydroxyethyl Caprylamide und/oder PPG-3 Hydroxyethyl Linoleamide bekannten Verbindungen enthält,
wobei sich die Mengenangaben auf das Gesamtgewicht der Reinigungszusammensetzungen beziehen.

Neben Transparenz, Rheologieverhalten und Reinigungsvermögen sollen die erfindungsgemäßen Zusammensetzungen weiterhin einen signifikanten Hautpflegeeffekt aufweisen.
Die Haut soll sich nach dem Abspülen der Zusammensetzungen weich, glatt und trocken anfühlen - ein auf der Haut haftender Ölfilm soll jedoch vermieden werden.
Durch die stabile Inkorporation des mindestens einen Öls a) mittels Einarbeitung der Komponenten b) und c) in eine tensidische Basis gelingt es bereits, den gewünschten Hautpflegeeffekt zu erzielen. Es wurde jedoch gefunden, dass sich die Pflegeeigenschaften, insbesondere die feuchtigkeitsspendenden und/oder die regenerierenden Eigenschaften der erfindungsgemäßen Zusammensetzungen noch weiter steigern lassen, wenn Ihnen weitere Pflegestoffe - vorzugsweise bestimmte feuchtigkeitsspendende Wirkstoffe d), Extrakte aus Kamille e) und/oder Allantoin f) hinzugefügt werden.

In einer weiteren bevorzugten Ausführungsform enthalten die erfindungsgemäßen Zusammensetzungen weiterhin mindestens einen feuchtigkeitsspendenden Wirkstoff d) in einem Gewichtsanteil von 0,5 bis 20,0 Gew.-% am Gesamtgewicht der Reinigungszusammensetzungen.

Geeignete feuchtigkeitsspendende Wirkstoffe d), die in den erfindungsgemäßen Zusammensetzungen verwendet werden können, dürfen die Transparenz und die Viskosität der Zusammensetzungen nicht negativ beeinträchtigen.
Als besonders bevorzugte Wirkstoffe d) haben sich die bereits an früherer Stelle genannten C₂-C₆-Polyole sowie Milchsäure, Hyaluronsäure, Alginsäure und die dermatologisch verträglichen Derivate dieser Säuren herausgestellt.

In einer weiteren bevorzugten Ausführungsform enthalten die erfindungsgemäßen Zusammensetzungen weiterhin mindestens einen Extrakt aus Kamille e) in einem Gewichtsanteil von 0,05 bis 3,0 Gew.-% am Gesamtgewicht der Reinigungszusammensetzungen.

Der Extrakt aus Kamille e) kann nach bekannten Herstellungsverfahren - beispielsweise durch Mahlen - aus allen Pflanzenteilen oder aus einzelnen Teilen der Kamille (wie Blätter, Blüten, Wurzeln), und gegebenenfalls anschließender Trocknung und/oder Extraktion gewonnen werden.

Die Extraktion findet üblicherweise unter erhöhten Temperaturen in einem Extraktor statt (beispielsweise Soxhlet). Dabei werden geeignete Lösungsmittel verwendet, je nachdem, ob die öl- oder wasserlöslichen Bestandteile der Pflanze(nteile) benötigt werden.
Als Extraktionsmittel zur Herstellung der genannten Pflanzenextrakte können beispielsweise Wasser, Alkohole sowie deren Mischungen verwendet werden. Als Alkohole kommen beispielsweise niedere Alkohole wie Ethanol und Isopropanol, insbesondere aber auch mehrwertige Alkohole wie Ethylenglykol, Propylenglykol, Butylenglykol, Glycerin und/oder Sorbitol in Frage, und zwar sowohl als alleiniges Extraktionsmittel als auch in Mischung mit Wasser. So haben sich beispielsweise Pflanzenextrakte auf Basis von Wasser/Alkohol(en) im Verhältnis 1 : 10 bis 10 : 1 als besonders geeignet erwiesen.
Für die erfindungsgemäßen Reinigungsmittel besonders geeignet sind aufgrund ihrer hervorragenden hautberuhigenden Wirkung Extrakte aus Kamillenblüten. Diese können ölbasiert, wässrig oder wässrig-alkoholisch sein. Entsprechende Extrakte sind in der Mischung mit dem jeweiligen Extraktionsmittel im Handel von verschiedenen Anbietern erhältlich, beispielsweise von den Firmen Cosmetochem, Symrise oder Croda.
Ganz besonders bevorzugt werden in den erfindungsgemäßen Zusammensetzungen wässrige, wässrig-alkoholische oder ölbasierte Kamillenblütenextrakte e) eingesetzt.

In einer weiteren bevorzugten Ausführungsform enthalten die erfindungsgemäßen Zusammensetzungen weiterhin Allantoin f) in einem Gewichtsanteil von 0,005 bis 0,5 Gew.-% am Gesamtgewicht der Reinigungszusammensetzungen.

Neben den zuvor genannten wesentlichen und fakultativen Bestandteilen können die erfindungsgemäßen kosmetischen Reinigungszusammensetzungen zur weiteren Steigerung der pflegenden Eigenschaften der Mittel weitere konditionierende Wirkstoffe enthalten, vorzugsweise hautkonditionierende Wirkstoffe aus der Gruppe der kationischen Polymere und/oder der Vitamine.

Geeignete kationische Polymere sind beispielsweise:
- quaternisierte Cellulose-Derivate, wie sie unter den Bezeichnungen Celquat® und Polymer JR® im Handel erhältlich sind,
- hydrophob modifizierte Cellulosederivate, beispielsweise die unter dem Handelsnamen SoftCat® vertriebenen kationischen Polymere,
- kationische Alkylpolyglycoside,
- kationiserter Honig, beispielsweise das Handelsprodukt Honeyquat® 50,
- kationische Guar-Derivate, wie insbesondere die unter den Handelsnamen Cosmedia®Guar N-Hance® und Jaguar® vertriebenen Produkte,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und - methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
   sowie die unter den Bezeichnungen
- Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Bevorzugt sind kationische Polysaccharidpolymere wie quaternisierte Cellulosepolymere, hydrophob modifizierte kationische Cellulosederivate und/oder kationische Guarderivate sowie polymere Dimethyldiallylammoniumsalze und deren Copolymere mit Estern und Amiden von Acrylsäure und Methacrylsäure. Besonders bevorzugt sind die unter den INCI-Bezeichnungen Guar Hydroxypropyltrimonium Chloride, Polyquaternium-10, Polyquaternium-37, Polyquaternium-67 Polyquaternium-72, Polyquaternium-6 und/oder Polyquaternium-7 bekannten kationischen Polymere; insbesondere bevorzugt ist Polyquaternium-7.
Die erfindungsgemäßen kosmetischen Reinigungszusammensetzungen können kationische Polymere - vorzugsweise Polyquaternium-7 - in einem Gewichtsanteil 0,01 bis 3,00 Gew.-%, besonders bevorzugt 0,05 bis 2,00 Gew.-% und insbesondere 0,10 bis 1,50 Gew.-% am Gesamtgewicht der Reinigungszusammensetzungen enthalten.

Unter geeigneten "Vitaminen" sind bevorzugt die folgenden Vitamine, Provitamine und Vitaminvorstufen sowie deren Derivate zu verstehen:
- Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
- Vitamin B: zur Vitamin B-Gruppe oder zu dem Vitamin B-Komplex gehören u. a.
   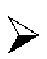 Vitamin B₁ (Thiamin)
   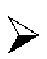 Vitamin B₂ (Riboflavin)
   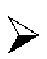 Vitamin B₃. Unter dieser Bezeichnung werden häufig die Verbindungen Nicotinsäure und Nicotinsäureamid (Niacinamid) geführt.
   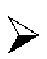 Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols, Pantolacton sowie kationisch derivatisierte Panthenole. Einzelne Vertreter sind beispielsweise das Panthenoltriacetat, der Panthenolmonoethylether und dessen Monoacetat sowie kationische Panthenolderivate.
   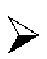 Vitamin B₆ (Pyridoxin sowie Pyridoxamin und Pyridoxal).
- Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol).
- Vitamin F: unter dem Begriff "Vitamin F" werden üblicherweise essentielle Fettsäuren, insbesondere Linolsäure, Linolensäure und Arachidonsäure, verstanden.
- Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Besonders bevorzugt sind Vitamine, Provitamine und Vitaminvorstufen aus den Gruppen A, B, E und H. Ganz besonders bevorzugt sind Nicotinsäureamid, Biotin, Tocopherol und/oder Panthenol; insbesondere bevorzugt sind Nicotinsäureamid und/oder Panthenol.

Der Gewichtsanteil des oder der Vitamins(e), Vitaminderivats(e), und/oder der Vitaminvorstufe(n) am Gesamtgewicht der erfindungsgemäßen kosmetischen Reinigungszusammensetzungen beträgt bevorzugt 0,005 bis 2,00 Gew.-%, mehr bevorzugt 0,006 bis 1,50 Gew.-%, besonders bevorzugt 0,008 bis 1,00 Gew.-% und insbesondere 0,01 bis 0,50 Gew.-%.

Weitere Wirk-, Hilfs- und Zusatzstoffe, die in den erfindungsgemäßen kosmetischen Reinigungszusammensetzungen eingesetzt werden können, sind beispielsweise:
- UV-Filter,
- Strukturanten wie Maleinsäure und Milchsäure,
- Farbstoffe zum Anfärben des Mittels,
- Substanzen zur Einstellung des pH-Wertes, beispielsweise α- und β-Hydroxycarbonsäuren wie Citronensäure, Milchsäure, Äpfelsäure, Glycolsäure,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Konservierungsmittel; insbesondere Natrium Benzoate,
- Viskositätsregler wie Elektrolytsalze (NaCl).

Ein zweiter Gegenstand der Erfindung ist die kosmetische Verwendung gemäß Anspruch 13 einer Kombination,
umfassend
neben mindestens einem Tensid und Wasser:
a) mindestens ein Öl,
b) mindestens einen Polyethylengruppen enthaltenden Lösungsvermittler, ausgewählt aus
   - Anlagerungsprodukten von 1 bis 20 Mol Ethylenoxid an Mono-, Di- und/oder Triester von Glycerin und linearen oder verzweigten, gesättigten oder ungesättigten C₈-C₂₄-Carbonsäuren und/oder
   - Anlagerungsprodukten von 25 bis 80 Mol Ethylenoxid an Rizinusöl und/oder gehärtetes Rizinusöl, und
c) mindestens ein Polyethylen- und/oder Polypropylengruppen enthaltendes Verdickungsmittel, ausgewählt aus
   - Anlagerungsprodukten von 1 bis 10 Mol Propylenoxid an C₈-C₂₄-Carbonsäureamide und/oder
   - Anlagerungsprodukten von 100 bis 300 Mol Ethylenoxid und/oder Propylenoxid an Mono-, Di-, Tri- und/oder Tetraester von Pentaerythrit an lineare oder verzweigte, gesättigte oder ungesättigte C₈-C₂₄-Carbonsäuren zur Herstellung transparenter rinse-off-Hautreinigungszusammensetzungen mit
   - verbesserter Pflegeleistung und
   - verbessertem Rheologieverhalten.

Für bevorzugte Ausführungsformen der erfindungsgemäßen Verwendung gilt mutatis mutandis das zu den erfindungsgemäßen Mitteln Gesagte.

### Beispiele:

Es wurden die folgenden erfindungsgemäßen transparenten kosmetischen Reinigungszubereitungen hergestellt (die Mengenangaben beziehen sich auf Gew.-%):

| | Formel 1 | Formel 2 | Formel 3 |
|---|---|---|---|
| anionisches Tensid | 5,00 - 15,00 | 6,00 - 11,00 | 6,50 - 10,00 |
| Öl | 0,10 - 2,00 | 0,30 - 1,80 | 0,40 - 1,70 |
| Anlagerungsprodukt von 1 bis 20 Mol Ethylenoxid an Mono-, Di- und/oder Triester von Glycerin und linearen oder verzweigten, gesättigten oder ungesättigten C₈-C₂₄-Carbonsäuren und/oder | 0,10 - 3,00 | 0,20 - 2,00 | 0,30 - 1,00 |
| Anlagerungsprodukt von 25 bis 80 Mol Ethylenoxid an Rizinusöl und/oder gehärtetes Rizinusöl | 0,50 - 5,00 | | |
| Anlagerungsprodukten von 1 bis 10 Mol Propylenoxid an C₈-C₂₄-Carbonsäureamide und/oder | 0,01 - 2,00 | 0,05 - 1,50 | 0,075 - 1,25 |
| Anlagerungsprodukten von 100 bis 300 Mol Ethylenoxid und/oder Propylenoxid an Mono-, Di-, Tri- und/oder Tetraester von Pentaerythrit an lineare oder verzweigte, gesättigte oder ungesättigte C₈-C₂₄-Carbonsäuren | | | |
| Wasser und ggfs. weitere Hilfs- und Zusatzstoffe | ad 100 | ad 100 | ad 100 |

| | Formel 4 | Formel 5 | Formel 6 |
|---|---|---|---|
| anionisches Tensid | 5,00 - 15,00 | 6,00 - 11,00 | 6,50 - 10,00 |
| Öl | 0,10 - 2,00 | 0,30 - 1,80 | 0,40 - 1,70 |
| Anlagerungsprodukt von 1 bis 20 Mol Ethylenoxid an Mono-, Di- und/oder Triester von Glycerin und linearen oder verzweigten, gesättigten oder ungesättigten C₈-C₂₄-Carbonsäuren und | 0,10 - 3,00 | 0,20 - 2,00 | 0,30 - 1,00 |
| Anlagerungsprodukt von 25 bis 80 Mol Ethylenoxid an Rizinusöl und/oder gehärtetes Rizinusöl | 0,50 - 5,00 | 0,75 - 4,00 | 1,00 - 3,00 |
| Anlagerungsprodukten von 1 bis 10 Mol Propylenoxid an C₈-C₂₄-Carbonsäureamide und/oder | 0,01 - 2,00 | 0,05 - 1,50 | 0,075 - 1,25 |
| Anlagerungsprodukten von 100 bis 300 Mol Ethylenoxid und/oder Propylenoxid an Mono-, Di-, Tri- und/oder Tetraester von Pentaerythrit an lineare oder verzweigte, gesättigte oder ungesättigte C₈-C₂₄-Carbonsäuren | | | |
| Wasser und ggfs. weitere Hilfs- und Zusatzstoffe | ad 100 | ad 100 | ad 100 |

| | Formel 7 | Formel 8 | Formel 9 |
|---|---|---|---|
| anionisches Tensid | 5,00 - 15,00 | 6,00 - 11,00 | 6,50 - 10,00 |
| Öl | 0,10 - 2,00 | 0,30 - 1,80 | 0,40 - 1,70 |
| Anlagerungsprodukt von 1 bis 20 Mol Ethylenoxid an Mono-, Di- und/oder Triester von Glycerin und linearen oder verzweigten, gesättigten oder ungesättigten C₈-C₂₄-Carbonsäuren | 0,10 - 3,00 | 0,20 - 2,00 | 0,30 - 1,00 |
| und/oder | | | |
| Anlagerungsprodukt von 25 bis 80 Mol Ethylenoxid an Rizinusöl und/oder gehärtetes Rizinusöl | | | |
| Anlagerungsprodukten von 1 bis 10 Mol Propylenoxid an C₈-C₂₄-Carbonsäureamide und | 0,01 - 2,00 | 0,05 - 1,50 | 0,075 - 1,25 |
| Anlagerungsprodukten von 100 bis 300 Mol Ethylenoxid und/oder Propylenoxid an Mono-, Di-, Tri- und/oder Tetraester von Pentaerythrit an lineare oder verzweigte, gesättigte oder ungesättigte C₈-C₂₄-Carbonsäuren | | | |
| Wasser und ggfs. weitere Hilfs- und Zusatzstoffe | ad 100 | ad 100 | ad 100 |

| | Formel 10 | Formel 11 | Formel 12 |
|---|---|---|---|
| anionisches Tensid | 5,00 - 15,00 | 6,00 - 11,00 | 6,50 - 10,00 |
| amphoteres Tensid | 2,00 - 15,00 | 3,00 - 9,00 | 3,50 - 8,00 |
| Öl | 0,10 - 2,00 | 0,30 - 1,80 | 0,40 - 1,70 |
| Anlagerungsprodukt von 1 bis 20 Mol Ethylenoxid an Mono-, Di- und/oder Triester von Glycerin und linearen oder verzweigten, gesättigten oder ungesättigten C₈-C₂₄-Carbonsäuren | 0,10 - 3,00 | 0,20 - 2,00 | 0,30 - 1,00 |
| und/oder | | | |
| Anlagerungsprodukt von 25 bis 80 Mol Ethylenoxid an Rizinusöl und/oder gehärtetes Rizinusöl | | | |
| Anlagerungsprodukten von 1 bis 10 Mol Propylenoxid an C₈-C₂₄-Carbonsäureamide und/oder | 0,01 - 2,00 | 0,05 - 1,50 | 0,075 - 1,25 |
| Anlagerungsprodukten von 100 bis 300 Mol Ethylenoxid und/oder Propylenoxid an Mono-, Di-, Tri- und/oder Tetraester von Pentaerythrit an lineare oder verzweigte, gesättigte oder ungesättigte C₈-C₂₄-Carbonsäuren | | | |
| Wasser und ggfs. weitere Hilfs- und Zusatzstoffe | ad 100 | ad 100 | ad 100 |

| | Formel 13 | Formel 14 | Formel 15 |
|---|---|---|---|
| anionisches Tensid | 5,00 - 15,00 | 6,00 - 11,00 | 6,50 - 10,00 |
| amphoteres Tensid | 2,00 - 15,00 | 3,00 - 9,00 | 3,50 - 8,00 |
| Öl | 0,10 - 2,00 | 0,30 - 1,80 | 0,40 - 1,70 |
| Anlagerungsprodukt von 1 bis 20 Mol Ethylenoxid an Mono-, Di- und/oder Triester von Glycerin und linearen oder verzweigten, gesättigten oder ungesättigten C₈-C₂₄-Carbonsäuren | 0,10 - 3,00 | 0,20 - 2,00 | 0,30 - 1,00 |
| und | | | |
| Anlagerungsprodukt von 25 bis 80 Mol Ethylenoxid an Rizinusöl und/oder gehärtetes Rizinusöl | 0,50 - 5,00 | 0,75 - 4,00 | 1,00 - 3,00 |
| Anlagerungsprodukten von 1 bis 10 Mol Propylenoxid an C₈-C₂₄-Carbonsäureamide | 0,01 - 2,00 | 0,05 - 1,50 | 0,075 - 1,25 |
| und | | | |
| Anlagerungsprodukten von 100 bis 300 Mol Ethylenoxid und/oder Propylenoxid an Mono-, Di-, Tri- und/oder Tetraester von Pentaerythrit an lineare oder verzweigte, gesättigte oder ungesättigte C₈-C₂₄-Carbonsäuren | | | |
| Wasser und ggfs. weitere Hilfs- und Zusatzstoffe | ad 100 | ad 100 | ad 100 |

| | Formel 16 | Formel 17 | Formel 18 |
|---|---|---|---|
| anionisches Tensid | 5,00 - 15,00 | 6,00 - 11,00 | 6,50 - 10,00 |
| amphoteres Tensid | 2,00 - 15,00 | 3,00 - 9,00 | 3,50 - 8,00 |
| Öl | 0,10 - 2,00 | 0,30 - 1,80 | 0,40 - 1,70 |
| PEG-7 Glyceryl Cocoate und/oder | 0,10 - 3,00 | 0,20 - 2,00 | 0,30 - 1,00 |
| PEG-40 Hydrogenated Castor Oil | 0,50 - 5,00 | 0,75 - 4,00 | 1,00 - 3,00 |
| Anlagerungsprodukten von 1 bis 10 Mol Propylenoxid an C₈-C₂₄-Carbonsäureamide | 0,01 - 2,00 | 0,05 - 1,50 | 0,075 - 1,25 |
| und | | | |
| Anlagerungsprodukten von 100 bis 300 Mol Ethylenoxid und/oder Propylenoxid an Mono-, Di-, Tri- und/oder Tetraester von Pentaerythrit an lineare oder verzweigte, gesättigte oder ungesättigte C₈-C₂₄-Carbonsäuren | | | |
| Wasser und ggfs. weitere Hilfs- und Zusatzstoffe | ad 100 | ad 100 | ad 100 |

| | Formel 19 | Formel 20 | Formel 21 |
|---|---|---|---|
| anionisches Tensid | 5,00 - 15,00 | 6,00 - 11,00 | 6,50 - 10,00 |
| amphoteres Tensid | 2,00 - 15,00 | 3,00 - 9,00 | 3,50 - 8,00 |
| Öl | 0,10 - 2,00 | 0,30 - 1,80 | 0,40 - 1,70 |
| PEG-7 Glyceryl Cocoate | 0,10 - 3,00 | 0,20 - 2,00 | 0,30 - 1,00 |
| und/oder | | | |
| PEG-40 Hydrogenated Castor Oil | 0,50 - 5,00 | 0,75 - 4,00 | 1,00 - 3,00 |
| PPG-2 Hydroxyethyl Cocamide | 0,01 - 2,00 | 0,05 - 1,50 | 0,075 - 1,25 |
| und/oder | | | |
| PEG-150 Pentaerythrityl tetrastearate | | | |
| Wasser und ggfs. weitere Hilfs- und Zusatzstoffe | ad 100 | ad 100 | ad 100 |

| | Formel 21 | Formel 22 | Formel 23 |
|---|---|---|---|
| anionisches Tensid | 5,00 - 15,00 | 6,00 - 11,00 | 6,50 - 10,00 |
| amphoteres Tensid | 2,00 - 15,00 | 3,00 - 9,00 | 3,50 - 8,00 |
| Öl | 0,10 - 2,00 | 0,30 - 1,80 | 0,40 - 1,70 |
| PEG-7 Glyceryl Cocoate | 0,10 - 3,00 | 0,20 - 2,00 | 0,30 - 1,00 |
| und | | | |
| PEG-40 Hydrogenated Castor Oil | 0,50 - 5,00 | 0,75 - 4,00 | 1,00 - 3,00 |
| PPG-2 Hydroxyethyl Cocamide | 0,01 - 2,00 | 0,05 - 1,50 | 0,075 - 1,25 |
| und | | | |
| PEG-150 Pentaerythrityl tetrastearate | | | |
| Wasser und ggfs. weitere Hilfs- und Zusatzstoffe | ad 100 | ad 100 | ad 100 |

| | Formel 24 | Formel 25 | Formel 26 |
|---|---|---|---|
| anionisches Tensid | 5,00 - 15,00 | 6,00 - 11,00 | 6,50 - 10,00 |
| amphoteres Tensid | 2,00 - 15,00 | 3,00 - 9,00 | 3,50 - 8,00 |
| Öl | 0,10 - 2,00 | 0,30 - 1,80 | 0,40 - 1,70 |
| PEG-7 Glyceryl Cocoate | 0,10 - 3,00 | 0,20 - 2,00 | 0,30 - 1,00 |
| und | | | |
| PEG-40 Hydrogenated Castor Oil | 0,25 - 2,50 | 0,40 - 2,00 | 0,60 - 1,50 |
| und | | | |
| PEG-60 Hydrogenated Castor Oil | 0,05 - 2,50 | 0,10 - 2,00 | 0,15-1,50 |
| PPG-2 Hydroxyethyl Cocamide | 0,01 - 2,00 | 0,05 - 1,50 | 0,075 - 1,25 |
| und | | | |
| PEG-150 Pentaerythrityl tetrastearate | | | |
| Wasser und ggfs. weitere Hilfs- und Zusatzstoffe | ad 100 | ad 100 | ad 100 |

| | Formel 27 | Formel 28 | Formel 29 |
|---|---|---|---|
| anionisches Tensid | 5,00 - 15,00 | 6,00 - 11,00 | 6,50 - 10,00 |
| amphoteres Tensid | 2,00 - 15,00 | 3,00 - 9,00 | 3,50 - 8,00 |
| Öl | 0,10 - 2,00 | 0,30 - 1,80 | 0,40 - 1,70 |
| PEG-7 Glyceryl Cocoate | 0,10 - 3,00 | 0,20 - 2,00 | 0,30 - 1,00 |
| und | | | |
| PEG-40 Hydrogenated Castor Oil | 0,50 - 5,00 | 0,75 - 4,00 | 1,00 - 3,00 |
| PPG-2 Hydroxyethyl Cocamide | 0,01 - 2,00 | 0,05 - 1,50 | 0,075 - 1,25 |
| und | | | |
| PEG-150 Pentaerythrityl tetrastearate | | | |
| Glycerin und/oder Sorbitol | 5,00 - 25,00 | 7,50 - 20,00 | 10,00 - 17,5 |
| Wasser und ggfs. weitere Hilfs- und Zusatzstoffe | ad 100 | ad 100 | ad 100 |

| | Formel 30 | Formel 31 | Formel 32 |
|---|---|---|---|
| anionisches Tensid | 5,00 - 15,00 | 6,00 - 11,00 | 6,50 - 10,00 |
| amphoteres Tensid | 2,00 - 15,00 | 3,00 - 9,00 | 3,50 - 8,00 |
| Öl | 0,10 - 2,00 | 0,30 - 1,80 | 0,40 - 1,70 |
| PEG-7 Glyceryl Cocoate | 0,10 - 3,00 | 0,20 - 2,00 | 0,30 - 1,00 |
| und | | | |
| PEG-40 Hydrogenated Castor Oil | 0,50 - 5,00 | 0,75 - 4,00 | 1,00 - 3,00 |
| PPG-2 Hydroxyethyl Cocamide | 0,01 - 2,00 | 0,05 - 1,50 | 0,075 - 1,25 |
| und | | | |
| PEG-150 Pentaerythrityl tetrastearate | | | |
| Glycerin und/oder Sorbitol | 5,00 - 25,00 | 7,50 - 20,00 | 10,00 - 17,5 |
| Chamomilla Reticula Flower Extract | 0,01 - 3,00 | 0,02 - 2,00 | 0,03 - 1, 0 |
| Allantoin | 0,005 - 0,5 | 0,01 - 0,40 | 0,02 - 0,30 |
| Wasser und ggfs. weitere Hilfs- und Zusatzstoffe | ad 100 | ad 100 | ad 100 |

| | 33 | 34 | 35 |
|---|---|---|---|
| **Sodium Laureth Sulfate** | 7,00 | 7,00 | 7,00 |
| **Cocamidopropylbetain** | 5,30 | 5,30 | 5,30 |
| **Sonnenblumenöl** | 0,20 | | |
| **Mandelöl** | | | 0,20 |
| **Cranberryöl** | 0,01 | | |
| **Parfumöl** | 1,20 | 1,20 | |
| **PEG-7 Glyceryl Cocoate** | 0,50 | 0,50 | 0,50 |
| **PEG-40 Hydrogenated Castor Oil** | 1,30 | 1,30 | 1,30 |
| **PEG-60 Hydrogenated Castor Oil** | 0,20 | 0,20 | 0,20 |
| **Versathix® LQ** | 0,50 | 0,40 | 0,45 |
| **Glycerin** | 6,00 | 6,00 | 6,00 |
| **Sorbitol** | 5,60 | 5,60 | 5,60 |
| **Polyquaternium-7** | 0,27 | 0,27 | |
| **Chamomilla Reticula Flower Extract** | 0,01 | | 0,01 |
| **Allantoin** | | 0,01 | 0,01 |
| **Panthenol** | 0,20 | | |
| **Tocopherol** | | 0,03 | |
| **Konservierungsmittel** | q.s. | q.s. | q.s. |
| **pH-Stellmittel (bis pH 4,5** - **5,5)** | q.s. | q.s. | q.s. |
| **Wasser** | ad 100 | ad 100 | ad 100 |

## Patentansprüche

1. Transparente kosmetische Reinigungszusammensetzung, enthaltend neben mindestens einem Tensid und Wasser:
a) mindestens ein Öl,
b) mindestens einen Polyethylengruppen enthaltenden Lösungsvermittler, ausgewählt aus
- Anlagerungsprodukten von 1 bis 20 Mol Ethylenoxid an Mono- Di- und/oder Triester von Glycerin und linearen oder verzweigten, gesättigten oder ungesättigten C₈-C₂₄-Carbonsäuren in einem Gewichtsanteil von 0,1 bis 3,0 Gew.-% am Gesamtgewicht der Reinigungszusammensetzung und
- Anlagerungsprodukten von 25 bis 80 Mol Ethylenoxid an Rizinusöl und/oder gehärtetes Rizinusöl in einem Gewichtsanteil von 0,5 bis 5,0 Gew.-% am Gesamtgewicht der Reinigungszusammensetzung und
c) mindestens ein Polyethylen- und/oder Polypropylengruppen enthaltendes Verdickungsmittel in einem Gewichtsanteil von 0,01 bis 2,00 Gew.-% am Gesamtgewicht der Reinigungszusammensetzung, ausgewählt aus
- Anlagerungsprodukten von 1 bis 10 Mol Propylenoxid an C₈-C₂₄-Carbonsäureamide und/oder
- Anlagerungsprodukten von 100 bis 300 Mol Ethylenoxid und/oder Propylenoxid an Mono-, Di-, Tri- und/oder Tetraester von Pentaerythrit an lineare oder verzweigte, gesättigte oder ungesättigte C₈-C₂₄-Carbonsäuren.

2. Reinigungszusammensetzung nach Anspruch 1, wobei das mindestens eine Tensid ausgewählt ist aus mindestens einem anionischen Tensid (i), vorzugsweise einer Mischung aus mindestens einem anionischen (i) und mindestens einem amphoteren / zwitterionischen Tensid (ii).

3. Reinigungszusammensetzung nach einem der vorherigen Ansprüche, enthaltend
- ein oder mehrere anionische Tenside (i) in einem Gewichtsanteil von 5,0 bis 15,0 Gew.-% am Gesamtgewicht der Reinigungszusammensetzung und/oder
- eine Mischung mindestens einem anionischen (i) und mindestens einem amphoteren / zwitterionischen Tensid (ii) in einem Gewichtsanteil von 7,0 bis 20,0 Gew.-% am Gesamtgewicht der Reinigungszusammensetzung.

4. Reinigungszusammensetzung nach einem der vorherigen Ansprüche, wobei das mindestens eine Öl a) ausgewählt ist aus Parfumölen und/oder pflanzlichen Ölen, und wobei das mindestens eine Öl a) in einem Gewichtsanteil von 0,1 bis 2,0 Gew.-% am Gesamtgewicht der Reinigungszusammensetzung eingesetzt wird.

5. Reinigungszusammehsetzung nach einem der vorherigen Ansprüche, enthaltend als Lösungsvermittler b)
- eine unter der INCI-Bezeichnung PEG-7 Glyceryl Cocoate bekannte Verbindung in einem Gewichtsanteil von 0,1 bis 3,0 Gew.-% am Gesamtgewicht der Reinigungszusammensetzung und
- eine unter der INCI-Bezeichnung PEG-40 Hydrogenated Castor Oil und/oder eine unter der INCI-Bezeichnung PEG-60 Hydrogenated Castor Oil bekannte Verbindung in einem Gewichtsanteil von 0,5 bis 5,0 Gew.-% am Gesamtgewicht der Reinigungszusammensetzung.

6. Reinigungszusammensetzung nach einem der vorherigen Ansprüche, enthaltend als Polyethylen- und/oder Polypropylengruppen enthaltendes Verdickungsmittel c)
- mindestens eine der unter den INCI-Bezeichnungen PEG-105 Pentaerythrityl monobehenate, PEG-105 Pentaerythrityl dibehenate, PEG-105 Pentaerythrityl tribehenate, PEG-105 Pentaerythrityl tetrabehenate, PEG-150 Pentaerythrityl monoisostearate, PEG-150 Pentaerythrityl diisostearate, PEG-150 Pentaerythrityl triisostearate, PEG-150 Pentaerythrityl tetraisostearate, PEG-130 Pentaerythrityl monostearate, PEG-130 Pentaerythrityl distearate, PEG-130 Pentaerythrityl tristearate, PEG-150 Pentaerythrityl tetrastearate, PEG-150 Pentaerythrityl monolaurate, PEG-150 Pentaerythrityl dilaurate, PEG-150 Pentaerythrityl trilaurate und/oder PEG-150 Pentaerythrityl tetralaurate bekannten Verbindungen, und/oder
- mindestens eine der unter den INCI-Bezeichnungen PPG-2 Hydroxyethyl Cocamide, PPG-1 Hydroxyethyl Caprylamide und/oder PPG-3 Hydroxyethyl Linoleamide bekannten Verbindungen.

7. Reinigungszusammensetzung nach Anspruch 6, enthaltend als Polyethylen- und/oder Polypropylengruppen enthaltendes Verdickungsmittel c)
- mindestens eine unter der INCI-Bezeichnung PEG-150 Pentaerythrityl Tetrastearate bekannte Verbindung und/oder
- mindestens eine unter der INCI-Bezeichnung PPG-2 Hydroxyethyl Cocamide bekannte Verbindung.

8. Reinigungszusammensetzung nach einem der vorherigen Ansprüche, enthaltend
- 7,0 bis 20,0 Gew.-% einer Mischung aus mindestens einem anionischen (i) und mindestens eines amphoteren / zwitterionischen Tensid (ii),
- 0,1 bis 2,0 Gew.-% mindestens eines Öls, vorzugsweise eines Parfumöls und/oder eines pflanzlichen Öls,
- 0,1 bis 3,0 Gew.-% mindestens eines Anlagerungsproduktes von 1 bis 20 Mol Ethylenoxid an Mono-, Di- und/oder Triester von Glycerin und linearen oder verzweigten, gesättigten oder ungesättigten C₈-C₂₄-Carbonsäuren,
- 0,5 bis 5,0 Gew.-% mindestens eines Anlagerungsproduktes von 25 bis 80 Mol Ethylenoxid an Rizinusöl und/oder gehärtetes Rizinusöl,
- 0,01 bis 2,00 Gew.-% mindestens einer der unter den INCI-Bezeichnungen PEG-105 Pentaerythrityl monobehenate, PEG-105 Pentaerythrityl dibehenate, PEG-105 Pentaerythrityl tribehenate, PEG-105 Pentaerythrityl tetrabehenate, PEG-150 Pentaerythrityl monoisostearate, PEG-150 Pentaerythrityl diisostearate, PEG-150 Pentaerythrityl triisostearate, PEG-150 Pentaerythrityl tetraisostearate, PEG-130 Pentaerythrityl monostearate, PEG-130 Pentaerythrityl distearate, PEG-130 Pentaerythrityl tristearate, PEG-150 Pentaerythrityl tetrastearate, PEG-150 Pentaerythrityl monolaurate, PEG-150 Pentaerythrityl dilaurate, PEG-150 Pentaerythrityl trilaurate und/oder PEG-150 Pentaerythrityl tetralaurate bekannten Verbindungen, und
- 0,01 bis 2,00 Gew.-% mindestens einer der unter den INCI-Bezeichnungen PPG-2 Hydroxyethyl Cocamide, PPG-1 Hydroxyethyl Caprylamide und/oder PPG-3 Hydroxyethyl Linoleamide bekannten Verbindungen,
wobei sich die Mengenangaben auf das Gesamtgewicht der Reinigungszusammensetzungen beziehen.

9. Reinigungszusammensetzung nach einem der vorherigen Ansprüche, enthaltend mindestens einen feuchtigkeitsspendenden Wirkstoff d) in einem Gewichtsanteil von 0,5 bis 20,0 Gew.-% am Gesamtgewicht der Reinigungszusammensetzung.

10. Reinigungszusammensetzung nach Anspruch 9, wobei der mindestens eine feuchtigkeitsspendende Wirkstoff d) ausgewählt ist aus C₂-C₆-Polyolen, Milchsäure, Hyaluronsäure und/oder Alginsäure sowie aus dermatologisch verträglichen Salzen und/oder Derivaten dieser Säuren.

11. Reinigungszusammensetzung nach einem der vorherigen Ansprüche, enthaltend mindestens einen Extrakt aus Kamille e), vorzugsweise einen wässrigen, wässrig-alkoholischen oder ölbasierten Kamillenblütenextrakt, in einem Gewichtsanteil von 0,05 bis 3,0 Gew.-% am Gesamtgewicht der Reinigungszusammensetzung.

12. Reinigungszusammensetzung nach einem der vorherigen Ansprüche, enthaltend zusätzlich Allantoin f) in einem Gewichtsanteil von 0,005 bis 0,5 Gew.-% am Gesamtgewicht der Reinigungszusammensetzung.

13. Kosmetische Verwendung einer Kombination, umfassend neben mindestens einem Tensid und Wasser:
a) mindestens ein Öl,
b) mindestens einen Polyethylengruppen enthaltenden Lösungsvermittler, ausgewählt aus
- Anlagerungsprodukten von 1 bis 20 Mol Ethylenoxid an Mono- Di- und/oder Triester von Glycerin und linearen oder verzweigten, gesättigten oder ungesättigten C₈-C₂₄-Carbonsäuren in einem Gewichtsanteil von 0,1 bis 3,0 Gew.-% am Gesamtgewicht der Reinigungszusammensetzung und
- Anlagerungsprodukten von 25 bis 80 Mol Ethylenoxid an Rizinusöl und/oder gehärtetes Rizinusöl in einem Gewichtsanteil von 0,5 bis 5,0 Gew.-% am Gesamtgewicht der Reinigungszusammensetzung und
d) mindestens ein Polyethylen- und/oder Polypropylengruppen enthaltendes Verdickungsmittel in einem Gewichtsanteil von 0,01 bis 2,00 Gew.-% am Gesamtgewicht der Reinigungszusammensetzung, ausgewählt aus
- Anlagerungsprodukten von 1 bis 10 Mol Propylenoxid an C₈-C₂₄-Carbonsäureamide und/oder
- Anlagerungsprodukten von 100 bis 300 Mol Ethylenoxid und/oder Propylenoxid an Mono-, Di-, Tri- und/oder Tetraester von Pentaerythrit an lineare oder verzweigte, gesättigte oder ungesättigte C₈-C₂₄-Carbonsäuren
zur Herstellung transparenter rinse-off-Hautreinigungszusammensetzungen mit
- verbesserter Pflegeleistung,
- verbesserter Stabilität und
- verbessertem Fließverhalten.

## Claims

1. A transparent cosmetic cleansing composition containing, in addition to at least one surfactant and water:
a) at least one oil,
b) at least one solubilizer that contains polyethylene groups, selected from
- addition products of 1 to 20 mol ethylene oxide to mono-, di- and/or triesters of glycerol and linear or branched, saturated or unsaturated C₈-C₂₄ carboxylic acids in a weight proportion of 0.1 to 3.0 wt.% with respect to the total weight of the cleansing composition and
- addition products of 25 to 80 mol ethylene oxide to castor oil and/or hydrogenated castor oil in a weight proportion of 0.5 to 5.0 wt.% with respect to the total weight of the cleansing composition and
c) at least one thickener that contains polyethylene groups and/or polypropylene groups, in a weight proportion of 0.01 to 2.00 wt.% with respect to the total weight of the cleansing composition, selected from
- addition products of 1 to 10 mol propylene oxide to C₈-C₂₄ carboxamides and/or
- addition products of 100 to 300 mol ethylene oxide and/or propylene oxide to mono-, di-, tri- and/or tetraesters of pentaerythritol to linear or branched, saturated or unsaturated C₈-C₂₄ carboxylic acids.

2. The cleansing composition according to claim 1, wherein the at least one surfactant is selected from at least one anionic surfactant (i), preferably a mixture of at least one anionic (i) and at least one amphoteric/zwitterionic surfactant (ii).

3. The cleansing composition according to one of the preceding claims, containing
- one or more anionic surfactants (i) in a weight proportion of 5.0 to 15.0 wt.% with respect to the total weight of the cleansing composition and/or
- a mixture of at least one anionic (i) and at least one amphoteric/zwitterionic surfactant (ii) in a weight proportion of 7.0 to 20.0 wt.% with respect to the total weight of the cleansing composition.

4. The cleansing composition according to one of the preceding claims, wherein the at least one oil a) is selected from perfume oils and/or vegetable oils, and wherein the at least one oil a) is used in a weight proportion of 0.1 to 2.0 wt.% with respect to the total weight of the cleansing composition.

5. The cleansing composition according to one of the preceding claims, containing as the solubilizer b)
- a compound known under the INCI name PEG-7 glyceryl cocoate in a weight proportion of 0.1 to 3.0 wt.% with respect to the total weight of the cleansing composition and
- a compound known under the INCI name PEG-40 hydrogenated castor oil and/or a compound known under the INCI name PEG-60 hydrogenated castor oil in a weight proportion of 0.5 to 5.0 wt.% with respect to the total weight of the cleansing composition.

6. The cleansing composition according to one of the preceding claims, containing as the thickener c) that contains polyethylene groups and/or polypropylene groups
- at least one of the compounds known under the INCI names PEG-105 pentaerythrityl monobehenate, PEG-105 pentaerythrityl dibehenate, PEG-105 pentaerythrityl tribehenate, PEG-105 pentaerythrityl tetrabehenate, PEG-150 pentaerythrityl monoisostearate, PEG-150 pentaerythrityl diisostearate, PEG-150 pentaerythrityl triisostearate, PEG-150 pentaerythrityl tetraisostearate, PEG-130 pentaerythrityl monostearate, PEG-130 pentaerythrityl distearate, PEG-130 pentaerythrityl tristearate, PEG-150 pentaerythrityl tetrastearate, PEG-150 pentaerythrityl monolaurate, PEG-150 pentaerythrityl dilaurate, PEG-150 pentaerythrityl trilaurate and/or PEG-150 pentaerythrityl tetralaurate, and/or
- at least one of the compounds known under the INCI names PPG-2 hydroxyethyl cocamide, PPG-1 hydroxyethyl caprylamide and/or PPG-3 hydroxyethyl linoleamide.

7. The cleansing composition according to claim 6, containing as the thickener c) that contains polyethylene groups and/or polypropylene groups
- at least one compound known under the INCI name PEG-150 pentaerythrityl tetrastearate and/or
- at least one compound known under the INCI name PPG-2 hydroxyethyl cocamide.

8. The cleansing composition according to one of the preceding claims, containing
- 7.0 to 20.0 wt.% of a mixture consisting of at least one anionic (i) and at least one amphoteric/zwitterionic surfactant (ii),
- 0.1 to 2.0 wt.% of at least one oil, preferably a perfume oil and/or a vegetable oil,
- 0.1 to 3.0 wt.% of at least one addition product of 1 to 20 mol ethylene oxide to mono-, di- and/or triesters of glycerol and linear or branched, saturated or unsaturated C₈-C₂₄ carboxylic acids,
- 0.5 to 5.0 wt.% of at least one addition product of 25 to 80 mol ethylene oxide to castor oil and/or hydrogenated castor oil,
- 0.01 to 2.00 wt.% of at least one of the compounds known under the INCI names PEG-105 pentaerythrityl monobehenate, PEG-105 pentaerythrityl dibehenate, PEG-105 pentaerythrityl tribehenate, PEG-105 pentaerythrityl tetrabehenate, PEG-150 pentaerythrityl monoisostearate, PEG-150 pentaerythrityl diisostearate, PEG-150 pentaerythrityl triisostearate, PEG-150 pentaerythrityl tetraisostearate, PEG-130 pentaerythrityl monostearate, PEG-130 pentaerythrityl distearate, PEG-130 pentaerythrityl tristearate, PEG-150 pentaerythrityl tetrastearate, PEG-150 pentaerythrityl monolaurate, PEG-150 pentaerythrityl dilaurate, PEG-150 pentaerythrityl trilaurate and/or PEG-150 pentaerythrityl tetralaurate, and
- 0.01 to 2.00 wt.% of at least one of the compounds known under the INCI names PPG-2 hydroxyethyl cocamide, PPG-1 hydroxyethyl caprylamide and/or PPG-3 hydroxyethyl linoleamide,
wherein the quantities stated relate to the total weight of the cleansing compositions.

9. The cleansing composition according to one of the preceding claims, containing at least one moisturizing active ingredient d) in a weight proportion of 0.5 to 20.0 wt.% with respect to the total weight of the cleansing composition.

10. The cleansing composition according to claim 9, wherein the at least one moisturizing active ingredient d) is selected from C₂-C₆ polyols, lactic acid, hyaluronic acid and/or alginic acid and from dermatologically acceptable salts and/or derivatives of these acids.

11. The cleansing composition according to one of the preceding claims, containing at least one extract of chamomile e), preferably an aqueous, aqueous-alcoholic or oil-based chamomile blossom extract, in a weight proportion of 0.05 to 3.0 wt.% with respect to the total weight of the cleansing composition.

12. The cleansing composition according to one of the preceding claims, additionally containing allantoin f) in a weight proportion of 0.005 to 0.5 wt.% with respect to the total weight of the cleansing composition.

13. The cosmetic use of a combination comprising, in addition to at least one surfactant and water:
a) at least one oil,
b) at least one solubilizer that contains polyethylene groups, selected from
- addition products of 1 to 20 mol ethylene oxide to mono-, di- and/or triesters of glycerol and linear or branched, saturated or unsaturated C₈-C₂₄ carboxylic acids in a weight proportion of 0.1 to 3.0 wt.% with respect to the total weight of the cleansing composition and
- addition products of 25 to 80 mol ethylene oxide to castor oil and/or hydrogenated castor oil in a weight proportion of 0.5 to 5.0 wt.% with respect to the total weight of the cleansing composition and
d) at least one thickener that contains polyethylene groups and/or polypropylene groups, in a weight proportion of 0.01 to 2.00 wt.% with respect to the total weight of the cleansing composition, selected from
- addition products of 1 to 10 mol propylene oxide to C₈-C₂₄ carboxamides and/or
- addition products of 100 to 300 mol ethylene oxide and/or propylene oxide to mono-, di-, tri- and/or tetraesters of pentaerythritol to linear or branched, saturated or unsaturated C₈-C₂₄ carboxylic acids
for producing transparent rinse-off skin cleansing compositions having
- improved care performance,
- improved stability and
- improved flow behavior.

## Revendications

1. Composition nettoyante cosmétique transparente qui contient en plus d'au moins un tensioactif et d'eau :
a) au moins une huile,
b) au moins un promoteur de solubilisation contenant des groupes polyéthylène, choisi parmi
- des produits d'addition de 1 à 20 moles d'oxyde d'éthylène sur des mono-, di- et/ou triesters de glycérol et des acides carboxyliques en C₈-C₂₄ linéaires ou ramifiés, saturés ou insaturés, dans une proportion pondérale de 0,1 à 3,0 % en poids par rapport au poids total de la composition nettoyante, et
- des produits d'addition de 25 à 80 moles d'oxyde d'éthylène sur de l'huile de ricin et/ou de l'huile de ricin durcie dans une proportion pondérale de 0,5 à 5,0 % en poids par rapport au poids total de la composition nettoyante, et
c) au moins un agent épaississant contenant des groupes polyéthylène et/ou polypropylène dans une proportion en poids de 0,01 à 2,00 % en poids par rapport au poids total de la composition nettoyante, choisi parmi
- des produits d'addition de 1 à 10 moles d'oxyde de propylène sur des amides d'acides carboxyliques en C₈-C₂₄, et/ou
- des produits d'addition de 100 à 300 moles d'oxyde d'éthylène et/ou d'oxyde de propylène sur des mono-, di-, tri- et/ou tétraesters de pentaérythritol sur des acides carboxyliques en C₈-C₂₄ linéaires ou ramifiés, saturés ou insaturés.

2. Composition nettoyante selon la revendication 1, dans laquelle l'au moins un tensioactif est choisi parmi au moins un tensioactif anionique (i), de préférence un mélange d'au moins un tensioactif anionique (i) et d'au moins un tensioactif amphotère/zwitterionique (ii).

3. Composition nettoyante selon l'une des revendications précédentes, contenant
- un ou plusieurs tensioactifs anioniques (i) dans une proportion pondérale de 5,0 à 15,0 % en poids par rapport au poids total de la composition nettoyante, et/ou
- un mélange d'au moins un tensioactif anionique (i) et d'au moins un tensioactif amphotère/zwitterionique (ii) dans une proportion pondérale de 7,0 à 20,0 % en poids par rapport au poids total de la composition nettoyante.

4. Composition nettoyante selon l'une des revendications précédentes, l'au moins une huile a) étant choisie parmi des huiles de parfum et/ou des huiles végétales, et l'au moins une huile a) étant utilisée dans une proportion pondérale de 0,1 à 2,0 % en poids par rapport au poids total de la composition nettoyante.

5. Composition nettoyante selon l'une des revendications précédentes, contenant comme promoteur de solubilisation b)
- un composé connu sous la dénomination INCI PEG-7 Glyceryl cocoate, dans une proportion pondérale de 0,1 à 3,0 % en poids par rapport au poids total de la composition nettoyante, et
- un composé connu sous la dénomination INCI PEG-40 Hydrogenated castor oil et/ou un composé connu sous la dénomination INCI PEG-60 Hydrogenated castor oil, dans une proportion pondérale de 0,5 à 5,0 % en poids par rapport au poids total de la composition nettoyante.

6. Composition nettoyante selon l'une des revendications précédentes, contenant, comme agent épaississant c) contenant des groupes polyéthylène et/ou polypropylène,
- au moins l'un des composés connus sous les dénominations INCI PEG-105 Pentaerythrityl monobehenate, PEG-105 Pentaerythrityl dibehenate, PEG-105 Pentaerythrityl tribehenate, PEG-105 Pentaerythrityl tetrabehenate, PEG-150 Pentaerythrityl monoisostearate, PEG-150 Pentaerythrityl diisostearate, PEG-150 Pentaerythrityl triisostearate, PEG-150 Pentaerythrityl tetraisostearate, PEG-130 Pentaerythrityl monostearate, PEG-130 Pentaerythrityl distearate, PEG-130 Pentaerythrityl tristearate, PEG-150 Pentaerythrityl tetrastearate, PEG-150 Pentaerythrityl monolaurate, PEG-150 Pentaerythrityl dilaurate, PEG-150 Pentaerythrityl trilaurate et/ou PEG-150 Pentaerythrityl tetralaurate, et/ou
- au moins l'un des composés connus sous les dénominations INCI PPG-2 Hydroxyethyl cocamide, PPG-1 Hydroxyethyl caprylamide et/ou PPG-3 Hydroxyethyl linoleamide.

7. Composition nettoyante selon la revendication 6, contenant, comme agent épaississant c) contenant des groupes polyéthylène et/ou polypropylène,
- au moins un composé connu sous la dénomination INCI PEG-150 Pentaerythrityl tetrastearate, et/ou
- au moins un composé connu sous la dénomination INCI PPG-2 Hydroxyethyl cocamide.

8. Composition nettoyante selon l'une des revendications précédentes, contenant
- 7,0 à 20,0 % en poids d'un mélange d'au moins un tensioactif anionique (i) et d'au moins un tensioactif amphotère/zwitterionique (ii),
- 0,1 à 2,0 % en poids d'au moins une huile, de préférence une huile de parfum et/ou une huile végétale,
- 0,1 à 3,0 % en poids d'au moins un produit d'addition de 1 à 20 moles d'oxyde d'éthylène sur des mono-, di- et/ou triesters de glycérol et des acides carboxyliques en C₈-C₂₄ linéaires ou ramifiés, saturés ou insaturés,
- 0,5 à 5,0 % en poids d'au moins un produit d'addition de 25 à 80 moles d'oxyde d'éthylène sur de l'huile de ricin et/ou de l'huile de ricin durcie,
- 0,01 à 2,00 % en poids d'au moins l'un des composés connus sous les dénominations INCI PEG-105 Pentaerythrityl monobehenate, PEG-105 Pentaerythrityl dibehenate, PEG-105 Pentaerythrityl tribehenate, PEG-105 Pentaerythrityl tetrabehenate, PEG-150 Pentaerythrityl monoisostearate, PEG-150 Pentaerythrityl diisostearate, PEG-150 Pentaerythrityl triisostearate, PEG-150 Pentaerythrityl tetraisostearate, PEG-130 Pentaerythrityl monostearate, PEG-130 Pentaerythrityl distearate, PEG-130 Pentaerythrityl tristearate, PEG-150 Pentaerythrityl tetrastearate, PEG-150 Pentaerythrityl monolaurate, PEG-150 Pentaerythrityl dilaurate, PEG-150 Pentaerythrityl trilaurate et/ou PEG-150 Pentaerythrityl tetralaurate, et
- 0,01 à 2,00 % en poids d'au moins l'un des composés connus sous les dénominations INCI PPG-2 Hydroxyethyl cocamide, PPG-1 Hydroxyethyl caprylamide et/ou PPG-3 Hydroxyethyl linoleamide,
les quantités indiquées se rapportant au poids total des compositions nettoyantes.

9. Composition nettoyante selon l'une des revendications précédentes, contenant au moins une substance active hydratante d) dans une proportion pondérale de 0,5 à 20,0 % en poids par rapport au poids total de la composition nettoyante.

10. Composition nettoyante selon la revendication 9, dans laquelle l'au moins une substance active hydratante d) est choisie parmi les polyols en C₂-C₆, l'acide lactique, l'acide hyaluronique et/ou l'acide alginique, ainsi que parmi des sels et/ou des dérivés dermatologiquement acceptables de ces acides.

11. Composition nettoyante selon l'une des revendications précédentes, contenant au moins un extrait de camomille e), de préférence un extrait aqueux, hydroalcoolique ou huileux de fleur de camomille, dans une proportion pondérale de 0,05 à 3,0 % en poids par rapport au poids total de la composition nettoyante.

12. Composition nettoyante selon l'une des revendications précédentes, contenant en outre de l'allantoïne f) dans une proportion pondérale de 0,005 à 0,5 % en poids par rapport au poids total de la composition nettoyante.

13. Utilisation cosmétique d'une association comprenant, en plus d'au moins un tensioactif et d'eau :
a) au moins une huile,
b) au moins un promoteur de solubilisation contenant des groupes polyéthylène, choisi parmi
- des produits d'addition de 1 à 20 moles d'oxyde d'éthylène sur des mono-, di- et/ou triesters de glycérol et des acides carboxyliques en C₈-C₂₄ linéaires ou ramifiés, saturés ou insaturés, dans une proportion pondérale de 0,1 à 3,0 % en poids par rapport au poids total de la composition nettoyante, et
- des produits d'addition de 25 à 80 moles d'oxyde d'éthylène sur de l'huile de ricin et/ou de l'huile de ricin durcie dans une proportion pondérale de 0,5 à 5,0 % en poids par rapport au poids total de la composition nettoyante, et
d) au moins un agent épaississant contenant des groupes polyéthylène et/ou polypropylène dans une proportion en poids de 0,01 à 2,00 % en poids par rapport au poids total de la composition nettoyante, choisi parmi
- des produits d'addition de 1 à 10 moles d'oxyde de propylène sur des amides d'acides carboxyliques en C₈-C₂₄, et/ou
- des produits d'addition de 100 à 300 moles d'oxyde d'éthylène et/ou d'oxyde de propylène sur des mono-, di-, tri- et/ou tétraesters de pentaérythritol sur des acides carboxyliques en C₈-C₂₄ linéaires ou ramifiés, saturés ou insaturés
pour préparer des compositions transparentes de nettoyage de la peau, pouvant être rincées, présentant
- une performance de soin améliorée,
- une stabilité améliorée, et
- un comportement d'écoulement amélioré.
